# EUROPEAN PATENT SPECIFICATION

(11) **EP 0 699 748 B1**
(45) Date of publication and mention of the grant of the patent: **07.08.2002**
(21) Application number: 95303211.7
(22) Date of filing: 12.05.1995
(51) Int. Cl.: C12N 1/19, C12N 9/88, C12N 15/81, C12C 11/02

(54) **Decreased hydrogen sulfide formation in beer production**
Verringerter Schwefelwasserstoffbindung bei der Bierherstellung
Formation d'hydrogène sulfuré réduite dans la production de la bière

(30) Priority: 13.05.1994 JP 9985594
(43) Date of publication of application: 06.03.1996
(73) Proprietor: SUNTORY LIMITED, Kita-ku, Osaka-shi, Osaka (JP)
(72) Inventor: Omura, Fumihiko, Minoh-shi, Osaka (JP); Shibano, Yuji, Toyonaka-shi, Osaka (JP); Fukui, Nobuyuki, Takasuki-shi, Osaka (JP); Nakatani, Kazuo, Nagaokakyo-shi, Kyoto (JP)
(74) Representative: Stoner, Gerard Patrick

(56) References cited:
- OMURA F. ET AL.: "Reduction of hydrogen sulfide production in brewing yeast by constitutive expression of MET25 gene." JOURNAL OF THE AMERICAN SOCIETY OF BREWING CHEMISTS, vol. 53, no. 2, 28 April 1995, pages 58-62, XP002066284
- TEZUKA H. ET AL.: "Cloning of a gene suppressing hydrogen sulfide production by Saccharomyces cerevisiae and its expression in a brewing yeast." JOURNAL OF THE AMERICAN SOCIETY OF BREWING CHEMISTS, vol. 50, 1992, pages 130-133, XP002066285
- KORCH C. ET AL.: "A mechanism for sulfite production in beer and how to increase sulfite levels by recombinant genetics." PROCEEDINGS OF THE EUROPEAN BREWERY CONVENTION CONGRESS, 1991, CONGRESS 23, LISBON, 1991, pages 201-208, XP002066286
- HANSEN J ET AL: "Modification of biochemical pathways in industrial yeasts" JOURNAL OF BIOTECHNOLOGY, vol. 49, no. 1, 20 August 1996, page 1-12 XP004037090
- BITTER G.A. & EGAN K.M.: "Expression of heterologous genes in Saccharomyces cerevisiae from vectors utilizing the glyceraldehyde-3-phosphate dehydrogenase promoter." GENE, vol. 32, 1984, pages 263-274, XP002066737

## Description

The present invention relates to yeast producing a reduced amount of hydrogen sulfide during the production of beer by constitutively expressing a structural gene for O-acetylhomoserine sulfhydrylase and a process for production of beer using said yeast.

Beer yeast (submerged yeast) used for production of light color beer produces hydrogen sulfide. The generation of hydrogen sulfide is one of the causes of immature beer odor which detracts from the quality of beer. To reduce the generation of hydrogen sulfide to be low the threshold level, postfermentation and elongation of the storage period have been carried out.

So far, to reduce the generation of hydrogen sulfide, research relating to factors effecting on the generation of hydrogen sulfide (Jangaard, N.O. et al., Amer, Soc. Brew. Chem. Proc. p 46, 1973; Kuroiwa, Y. et al., Brew. Dig., 45, 44, 1970; Hysert, D.W. et al., J. Amer. Soc. Brew. Chem., 34, 25, 1976), research for breeding of yeast having reduced productivity of hydrogen sulfide by mutagenesis and cell fusion has been reported (Molzahm, S.W.; J. Amer. Soc. Brew. Chem. 35, 54, 1977).

Since these methods not only reduce the generation of hydrogen sulfide by yeast, but also affect other brewing characteristics such as the fermentation rate, and the flavor of beer, then yeast suitable for brewing beer has not been obtained. Recently, breeding of brewery yeast using genetic engineering has been started, and Japanese Unexamined Patent Publication (Kokai) No. 5-244955 discloses that beer yeast into which a DNA fragment coding for systathionine β-synthase has been introduced reduces the generation of hydrogen sulfide. However, the extent of the reduction is small, and the amount of hydrogen sulfide generated by transformant is 60 to 80% of that by parent strain.

In the metabolism of yeast, hydrogen sulfide is generated by the reduction of sulfate anion SO₄⁻⁻ uptaken from a medium.

This metabolic process is a biosynthesis pathway for sulfur-containing amino acids such as methionine, cysteine etc., and enzymes and genes therefor for each step have been reported in detail (Tabor, H and Tabor, C.w. (eds.); Methods in Enzymology Vol. 17B, Academic Press, London, 1971; Jakoby, W.B. and Griffith, O.W. (eds.); Methods in Enzymology Vol. 143, Academic Press, London, 1987).

O-Acetylhomoserine sulfhydrylase is an enzyme which transfers a sulfur atom from hydrogen sulfide to O-acetylhomoserine, and encoded by MET25 gene. This enzyme also has an activity to transfer a sulfur atom to O-acetylserine. In any event, since O-acetylhomoserine sulfhydrylase is an enzyme using hydrogen sulfide as a substrate, it is expected that an increase of the activity in said enzyme in yeast cells results in an increase of consumption of the substrate hydrogen sulfide, and a decrease in an amount of hydrogen sulfide generated.

A general proposal made herein is therefore to decrease the presence of hydrogen sulfide in a beer brewing process by adapting a gene (such as MET25) coding for yeast O-acetylhomoserine sulfhydrylase so that the gene is expressed, preferably constitutively, in a brewing yeast.

In specific aspects we provide yeast belonging to the genus Saccharomyces transformed with a recombinant vector comprising a promoter from a gene constitutively expressed in yeast, located upstream of a structural gene coding for yeast O-acetylhomoserine sulfhydrylase, and capable of a constitutive expression of a large amount of O-acetylhomoserine sulfhydrylase resulting in a decrease of hydrogen sulfide generated in a beer brewing process; a process for production of beer using the above-mentioned yeast, and a process of making the special yeast itself.

Figure 1 shows a process for the isolation of an MET25 gene by PCR and the construction of plasmid pUC/MET25 containing the MET25 gene.

Fig. 2 shows a process for the construction of plasmid pYGl, wherein the symbol G418^{r} represents a gene providing resistance against G418 under the control by a promoter of GAPDH gene; PGAPDH represents a promoter of GAPDH gene; Ap^{r} represents ampicillin resistance gene; TRP1 represents a gene providing tryptophan prototrophy; and IR1 represents reversed repeat sequence.

Fig. 3 shows a process for the construction of plasmid pEG25.

Fig. 4 is a graph showing the change of an amount of hydrogen sulfide generated by a yeast strain transformed with MET25 gene for 200 hours. The open circles show a result for the parental strain BH84, and the solid circles show a result for the transformant BH M38-2 strain.

Fig. 5 is a graph showing an amount (OD660) of yeast grown during fermentation in Example 6.

Fig. 6 is a graph showing a course of consumption of extract during fermentation in Example 6.

### DETAILED DESCRIPTION

Saccharomyces cerevisiae generates hydrogen sulfide by reducing sulfate anion in a culture medium or wort when methionine or cysteine is biosynthesized in cells. O-acetylhomoserine sulfhydrylase, which is a MET25 gene product, uses hydrogen sulfide as a substrate and biosynthesizes homocysteine and cysteine, and expression of the MET25 gene is repressed by methionine in culture medium.

The present inventors amplified and isolated MET25 gene from chromosomal DNA of Saccharomyces cerevisiae X2180-1A using polimerase chain reaction (PCR), and constructed a plasmid comprising a promoter of a gene constitutively expressed in yeast, positioned upstream of the structural gene portion of said MET25 gene. The resulting plasmid DNA was introduced into yeast cells, and the bred strains which constitutively express the MET25 gene were obtained. When generation of hydrogen sulfide was tested during beer brewing, the generation of hydrogen sulfide was decreased to 2% for the bred strain in comparison with that for the parent strain.

As host yeast for the above-mentioned process, brewery yeast, for example, beer yeast such as Saccharomyces cerevisiae BH84, IFO 1951, IFO 1952, IFO 1953, IFO 1954, available from Institute for Fermentation Osaka, 17-85, Juso-honmachi 2-chome, Yodogawa-ku, Osaka 532, Japan, can be used. As promoters for constitutive expression, any constitutive promoter of yeast origin, for example, promoter of glycelaldehyde-3-phosphate dehydrogenase (GAPDH) gene, promoter of 3-phosphoglycerate kinase (PGK) gene, and the like can be used. The PGK gene has been cloned, described for example by Tuite, M.F. et al., EMBO J. Vol. 1, p 603 (1982), and can be easily obtained according to a conventional procedure.

As a vector used for introduction of a gene into yeast cells, any of multicopy-type (YEp type) vector, single-type (YCp type) vector and chromosome-integrating type (YIp type) vector can be used. For example, YEp51 (Broach J.R. et al., Experimental Manipulation of Gene Expression, Academic Press, New York, 1983, p 83) is known as YEp type vector; YCp50 (Rose, M.D. et al., Gene, Vol. 60, p 237 (1987)) is known as a YCp type vector; and YIp5 (Struhl K. et al., Proc. Natl, Acad. Sci. USA, Vol. 76, p 1035 (1979)) is known as a YIp type vector, and all of them can be easily obtained.

As selection marker for transformation, auxotropic marker cannot be used for brewery yeast, therefore G418 resistance gene (G418^{r}), cupper resistance gene (CUP1) (Karin, M. et al., Proc. Natl. Acad. Sci. USA, Vol. 81, p 337 (1984)), serulenine resistance gene (fas2m, PDR4) (J. Inokoshi et al., Seikagaku, Vol. 64, p 660 (1992); Hussain, M. et al., Gene Vol. 101, p 149 (1991)), and the like are used.

A promoter of a constitutively expressed gene and a structural gene for O-acetylhomoserine sulfhydrylase under the control by said promoter are introduced into beer yeast cells, and a high level of expression of O-acetylhomoserine sulfhydrylase is maintained in yeast. As a result, since O-acetylhomoserine sulfhydrylase rapidly consumes hydrogen sulfide, then the amount of hydrogen sulfide generated and a concentration of hydrogen sulfide in beer are decreased to a low level.

### EXAMPLES

The present invention is further explained in detail by Examples, though the present invention should not be limited thereto.

### Example 1. Cloning of MET25 gene

The MET25 gene of yeast Saccharomyces cerevisiae has already been cloned and its nucleotide sequence has been reported (Kerjan, P. et al., Nucl. Acids Res. 14, 7861, 1986). Based on this information, the MET25 gene was amplified by PCR and isolated. Chromosomal DNA was extracted from a yeast for research, the strain X2180-1A (University of California at Berkey, Yeast Genetic Stock Center), and used as template for PCR.

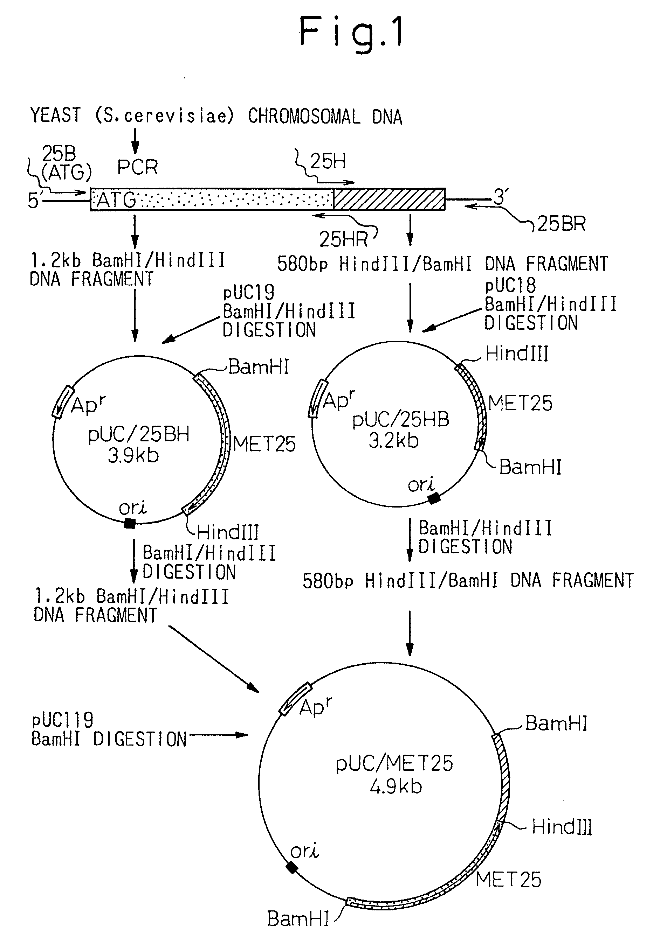

As primers for PCR, synthetic oligonucleotides 258 (ATG) (5'-CTGGATCCCCCATCCATACAATGCCAT-3') (SEQ ID NO: 1), 25HR(5'-GAGGCAAGCTTTAAATTGTC-3') (SEQ ID NO: 2), 25H(5'-GACAATTTAAAGCTTGCCTC-3') (SEQ ID NO: 3), and 25BR(5'-CCGGATCCCGCGAAGTTTTCCTGATTT-3') (SEQ ID NO: 4) were used. A combination of the primers 25B(ATG) and 25HR amplified the nucleotide numbers -14 to 1120 of the MET25 gene and provided BamHI-Hind III DNA fragment of about 1.2 kb; and a combination of the primers 25H and 25BR amplified the nucleotide numbers 1121 to 1712 of the MET25 gene and provided a Hind III-BamHI fragment of about 580 bp (Fig. 1).

Since the synthetic DNA was provided with restriction enzyme sites, the amplified DNA can be digested with restriction enzymes, and can be cloned into a conventional cloning vector. The BamHI-Hind III fragment of about 1.2 kb was inserted into an E. coli plasmid pUC19 (Takara Shuzo) to construct a plasmid pUC/25BH, and the Hind III-BamHI fragment of about 580 bp was inserted into plasmid pUC18 (Takara Shuzo) to construct a plasmid pUC/25HB (Fig. 1).

The nucleotide sequence of the MET25 gene thus obtained was tested by Sanger's method (Sanger, F., Science, 214, 1205, 1981), and confirmed to be the same as the reported sequence of the MET25 gene. MET25 gene portions were again removed from pUC25BH and pUC25HB using BamHI and Hind III, and the resulting DNA fragments were cloned into pUC119 plasmid (Takara Shuzo) so as to obtain an entire MET25 gene (pUC/MET25) (Fig. 1).

### Example 2. Construction of plasmid pYGI

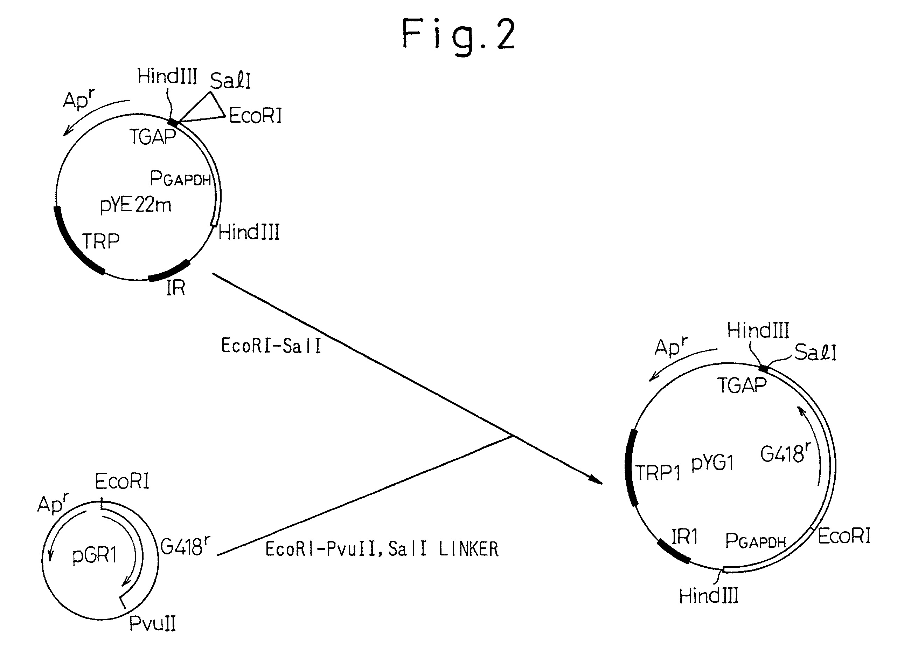

Plasmid pGRl containing a structural gene for G418 resistance was obtained according to a procedure described in Agric. Biol. Chem. Vol. 54, No. 10, p 2689 - 2696 (1990), and cleaved with EcoRI and PvuII to obtain a DNA fragment comprising a structural gene for G418 resistance. Sail linker was added to the DNA fragment, the resulting DNA fragment was cleaved with EcoRI and Sail, and the resulting DNA fragment was inserted into between a promoter and a terminator of GAPDH gene of pYE22m, to obtain pYGl (Fig. 2). Note that a process for construction of the plasmid is reported in Japanese Unexamined Patent Publication (Kokai) (No. 4-228078).

### Example 3. Construction of plasmid pEO25

Plasmid pEG25 is a YEp type plasmid having an origin of replication of yeast 2 µm plasmid (Baggs. J.D., Nature, 275, 104, 1978), capable of self-replication in yeast, having the MET25 gene coding for O-acetylhomoserine sulfhydrylase linked to a promoter of glycelaldehydephosphate dehydrogenase (GAPDH) (Holland, J.P. et al., J. Biol. Chem. 254, 9839, 1976), and further having as transformation makers TRP1 gene and G418 resistance gene (Oka, A. et al., J. Mol. Biol. 147, 217, 1981).

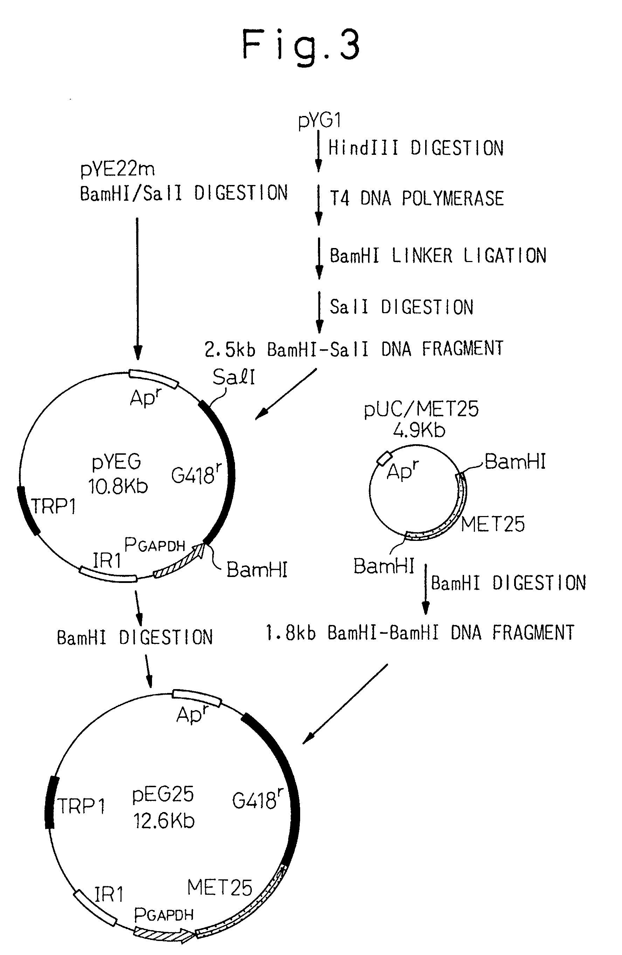

More specifically, plasmid pYGl was cleaved with Hind III and SalI to obtain a DNA fragment comprising 418 resistance gene of about 2.5 kb, which was then bluntended at the Hind III end with T4 DNA polymerase (Takara Shuzo), and BamHI-ended by addition of a BamHI linker. The resulting DNA fragment was inserted in between BamHI and SalI sites of pYE 22m, so as to construct pYEG (Fig. 3).

The plasmid pYEG thus obtained was cleaved with BamHI, and the cleaved plasmid was ligated with a BamHI fragment of about 1.8 kb comprising MET25 gene prepared from plasmid pUC/MET25 described in Example 1 to obtain pEQ 25 (Fig. 3).

### Example 4. Transformation of yeast

Cells of beer yeast BH84 strain (Technical University Munich; Veirhenstephen, 164 (stored strain No. 164) were treated with lithium acetate, and the plasmid solution and polyethylene glycol 4000 were added thereon. The mixture was incubated at 30°C for 30 minutes and 42°C for 5 minutes, and 2 ml of YPD liquid medium (yeast extract 1%, polypepton 2%, glucose 2%) were added thereon. The mixture was shaken at 30°C for 6 hours, and centrifuged to collect the cells. The resulting cells were plated on YPD plate medium (YPD liquid medium +2% agar) containing 300 ppm G418, and cultured at 30°C for 3 to 5 days. Colonies grown on the plate were picked up, and a transformed yeast was designated BH M38-2.

### Example 5. Analysis of amount of expression of mRNA for MET25 in transformant

The parental strain BH84 and the transformant BH M38-2 were separately cultured in 10 ml of Yeast Nitrogen Base (Difco) containing 2% glucose, and Yeast Nitrogen Base containing 2% glucose and 5 mM methionine, at 30°C for 17 hours. The cultured yeast cells were collected, washed and suspended in 0.2 ml of LETS buffer (0.1M LiCl, 1% (W/V) SDS, 0.2M Tris-HCl (pH 7.4), 0.01M EDTA), 0.4g of glass beads were added to the suspension, and the cells were disrupted with a bead beater for 3 minutes.

The cell disruptant thus obtained was centrifuged at 10,000 × g for 10 minutes to obtain a supernatant, which was then treated three times with phenol. Ethanol was added thereto and a total RNA was precipitated at -20°C. After centrifugation, the precipitate was rinsed with 70% ethanol and concentrated to dryness. The dried precipitate was dissolved in distilled water to obtain a total RNA solution. 40 µg of the total RNA thus obtained was developed by formaldehyde gel electrophoresis according to a conventional procedure, and transferred to a membrane. The membrane was subjected to hybridization with a probe i.e., a DNA fragment comprising MET25 gene labeled with ³²P.

The strength of expression was measured using an imaging plate (Fuji Film, BAS 2000). The result is shown in Table 1 taking a value for the parental BH84 strain in the absence of methionine as 100%.

**Table 1**

| Amount of expression of MET25 mRNA | | |
|---|---|---|
| Yeast strain | Absence of methionine | Presence of methionine |
| BH84 | 100% | 10.2% |
| BH M38-2 | 295.3% | 413.2% |

For the parental strain BH84, expression of MET25 gene was repressed by methionine in a culture medium, while the transformant BH M38-2 expressed MET25 mRNA by an amount 3 to 4 times higher than the parent regardless the presence and absence of methionine.

### Example 6. Analysis of an amount of hydrogen sulfide released during beer brewing

The parental strain BH84 and the transformant BH M38-2 were tested for fermentation under the condition described in Table 2. 10 ppm of a selection agent G418 was added to wort for only the strain BH M38-2.

**Table 2**

| | |
|---|---|
| Concentration of extract in wort | 11% |
| Volume of wort | 2L |
| Concentration of oxygen dissolved in wort | 9 ppm |
| Fermentation temperature | 12°C constant |
| Yeast inoculated | 10 g wet cell/2L |

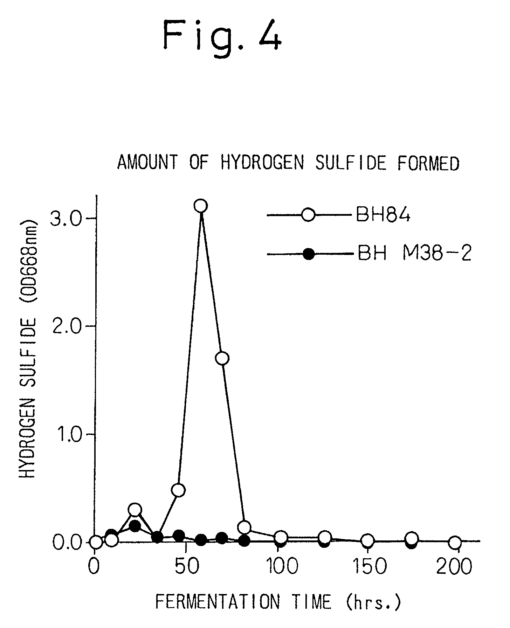

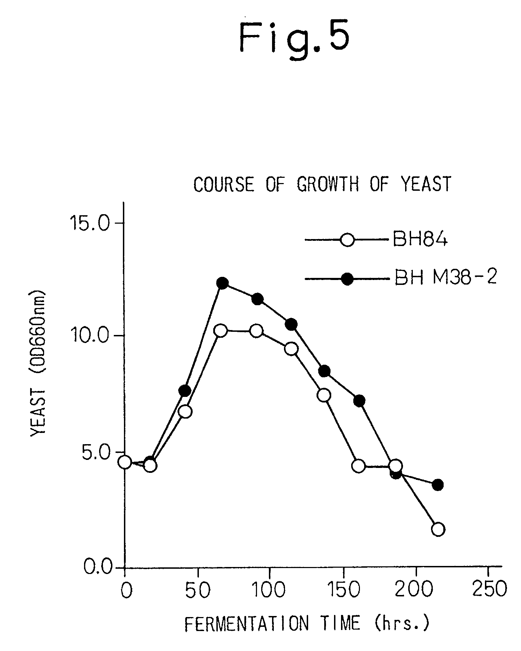

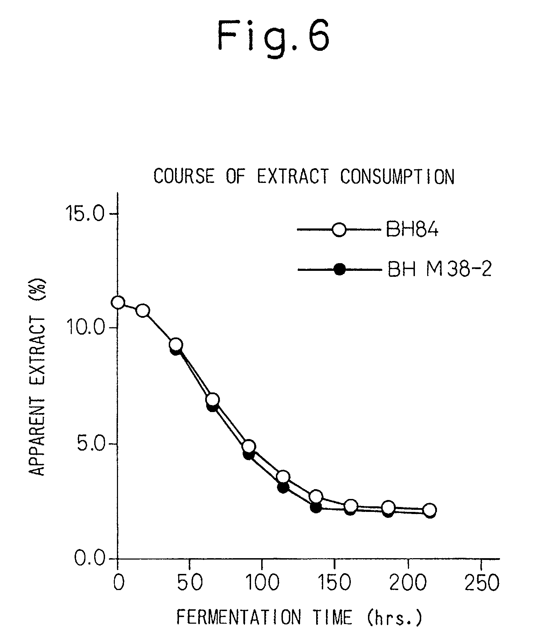

Quantitation of hydrogen sulfide was carried out by trapping hydrogen sulfide in fermentation gas with a zinc acetate solution, generating methylene blue and measuring absorbance at OD668 (Jangaard, N.O., et al., Amer. Soc. Brew. Chem. Proc. p 46, 1973) (Fig. 4). In addition, the growth of yeast (OD660) during fermentation (Fig. 5) and course of extract consumption (Fig. 6) were observed, and components of beer were analyzed (Table 3).

As a result, as seen from Fig. 4, the amount of hydrogen sulfide generated in 200 hours by the transformant BH M38-2 to which the MET25 gene had been introduced was decreased to about 2% of that of the parental strain. On the other hand, the growth of yeast was normal (Fig. 5), and the course of fermentation by the transformant was not different from that of the parent strain (Fig. 6). In addition, the result of analysis of beer components was not different from that of the parent strain, except that an amount of SO₂ was decreased in the transformant (Table 3). In addition, abnormal odor was not detected in the bred strain in a sensory test.

**Table 3**

| Analysis of beer components | | |
|---|---|---|
| Item of Analysis | BH84 | BH M38-2 |
| Original extract (w/w%) | 11.89 | 11.79 |
| Apparent extract (w/w%) | 1.97 | 1.97 |
| Real extract (w/w%) | 3.87 | 3.85 |
| Alcohol (w/w%) | 4.14 | 4.09 |
| Fermentation ratio (%) | 83.4 | 83.3 |
| Color | 6.6 | 6.7 |
| pH | 4.55 | 4.54 |
| Bitter value (BUs) | 23.9 | 24.3 |
| Total nitrogen (mg/100 ml) | 69.2 | 68.6 |
| Amino nitrogen (mg/100 ml) | 8.4 | 8.6 |
| Total polyphenol (ppm) | 125 | 132 |
| SO₂ (ppm) | 8.3 | 5.4 |
| Low volatile compounds (ppm) | | |
| Acetaldehyde | 1.4 | 1.3 |
| Ethyl acetate | 30.6 | 32.6 |
| n-Propanol | 9.2 | 10.2 |
| i-Butanol | 15.5 | 15.2 |
| Amyl acetate | 1.8 | 1.9 |
| Amylalcohol | 60.5 | 60.4 |

As seen from the above, it was confirmed that the present technique decreases hydrogen sulfide generated during fermentation process, and as a result,
is effective to improve the quality of beer, and shortening of post fermentation and storage period.

## Claims

1. A recombinant yeast belonging to the genus Saccharomyces having a promoter of a constitutively expressed gene and a structural gene coding for 0-acetylhomoserine sulfhydrylase (MET25) under the control by said promoter so that the recombinant yeast produces an amount of hydrogen sulfide lower than an amount of hydrogen sulfide produced by the parental yeast from which the recombinant yeast is derived.

2. A recombinant yeast according to claim 1 wherein the promoter of the constitutively expressed gene is a promoter of a glyceraldehyde-3-phosphate dehydrogenase (GAPDH) gene or a promoter of 3-phosphoglycerate kinase (PGK) gene.

3. A process for production of beer using a recombinant yeast according to claim 1 or 2, wherein the content of hydrogen sulfide in beer is decreased and the fermentation time is shortened in comparison with those of the parental yeast from which the recombinant yeast is derived.

## Patentansprüche

1. Rekombinante Hefe, die zur Gattung Saccharomyces gehört, mit einem Promotor eines konstitutiv ausgeprägten Gens und einem Strukturgen, das unter der Kontrolle des Promotors O-Acetylhomoserin-Sulfhydrylase kodiert (MET25), so daß die rekombinante Hefe Schwefelwasserstoff in einer geringeren Menge als die Schwefelwasserstoffmenge erzeugt, die von der Elternhefe erzeugt wird, von der die rekombinante Hefe stammt.

2. Rekombinante Hefe nach Anspruch 1, wobei der Promotor für das konstitutiv ausgeprägte Gen ein Promotor eines Glyceraldehyd-3-phosphat-Dehydrogenase(GAPDH)-Gens oder ein Promotor des 3-Phosphoglycerat-Kinase(PGK)-Gens ist.

3. Verfahren zur Herstellung von Bier unter Verwendung einer rekombinanten Hefe nach Anspruch 1 oder 2, wobei im Vergleich mit der Elternhefe, von der die rekombinante Hefe stammt, der Schwefelwasserstoffgehalt im Bier verringert und die Fermentationszeit verkürzt werden.

## Revendications

1. Une levure recombinante appartenant au genre Saccharomyces ayant un promoteur d'un gène exprimé de manière constitutive et un gène de structure codant pour la O-acétylhomosérine sulfhydrylase (MET25) sous le contrôle dudit promoteur de façon à ce que la levure recombinante produise une quantité de sulfure d'hydrogène inférieure à la quantité de sulfure d'hydrogène produite par une levure parentale dont la levure recombinante dérive.

2. Une levure recombinante selon la revendication 1, dans laquelle le promoteur du gène exprimé de manière constitutive est un promoteur d'un gène glycéraldéhyde-3-phosphate déshydrogénase (GAPDH) ou un promoteur d'un gène 3-phosphoglycérate kinase (PGK).

3. Un procédé pour produire une bière en utilisant une levure recombinante selon la revendication 1 ou 2, dans lequel la teneur en sulfure d'hydrogène dans la bière est réduite et le temps de fermentation est écourté en comparaison avec ceux de la levure parentale dont la levure recombinante dérive.
